# EUROPEAN PATENT SPECIFICATION

(11) **EP 2 027 842 B1**
(45) Date of publication and mention of the grant of the patent: **12.12.2012**
(21) Application number: 08014070.0
(22) Date of filing: 06.08.2008
(51) Int. Cl.: A61F 13/15

(54) **Absorbent product**
Saugfähiges Produkt
Produit absorbant

(30) Priority: 20.08.2007 JP 2007213494
(43) Date of publication of application: 25.02.2009
(73) Proprietor: Livedo Corporation, Shikoku-Chuo-shi, EHIME 799-0122 (JP)
(72) Inventor: Tatsukawa, Akiko, Mima-gun Tokushima 779-4104 (JP)
(74) Representative: Müller-Boré & Partner Patentanwälte

(56) References cited:
- EP-A- 0 945 111
- EP-A- 1 101 476
- GB-A- 2 296 445
- US-B1- 6 706 029

## Description

The present invention relates to an absorbent product for receiving excrement from a wearer.

In an absorbent product such as a light incontinence pad attached on a disposal diaper, underpants or the like, conventionally, a pair of side wall parts (so-called standing gathers) which stand toward a wearer on both sides in a width direction are provided for preventing leakage of excrement such as urine.

Japanese Patent Application Laid-Open No. 2001-25485 (Document 1) and Japanese Patent Application Laid-Open No. 2001-137282 (Document 2) relate to an absorbent product having a standing cuff for portions around legs of wearer. The standing cuff has a standing part standing toward the center of the absorbent product and a plane part spreading outward from an upper end of the standing part. In the absorbent product, the plane parts of the standing cuffs are made to fit the portions around the legs of the wearer for preventing leakage of urine from the sides of the absorbent product.

In the absorbent products of Documents 1 and 2, since the plane part is formed by folding outward a portion continuous from the standing part of the standing cuff, the thickness of the plane part is equal to that of the standing part and is relatively thin. For this reason, the plane part deforms and is partly away from the portions around the legs of the wearer and there is a possibility that urine or the like leaks from a small space between the plane part and the portions around the legs. Further, an elastic member provided between a folded part and an outer edge of the plane part tightly fits the wearer and feeling of the wearer is decreased in wearing the absorbent product.

GB-A-2 296 445 describes an absorbent article, comprising an absorbent member disposed between a liquid permeable topsheet and an impermeable backsheet, and lateral flaps extending from opposite sides, the flaps being folded to form anti-leakage walls and, outwardly of such walls, anti-leakage surfaces.

EP-A-0 945 111 discloses a disposable diaper including barrier cuffs around the wearer's legs, wherein each of the barrier cuffs longitudinally extends on an inner surface of the disposable diaper and includes a supporting wall section adapted to rise on the inner surface of the diaper, and a sealing surface zone which includes a first and a second overhead section, extending inwards and outwards, respectively, from the supporting wall section.

US-B1-6 706 029 discloses a diaper structure which is formed with a leakage preventing arrangement in the form of a flexible flap located outwardly with respect to a diaper absorbent core.

The present invention is intended for an absorbent product for receiving excrement from a wearer. It is an object of the present invention to improve stability of the shape of a plane part in a pair of side wall parts in the absorbent product and to enhance fitting to the wearer.

Accordingly, the present invention provides an absorbent product as defined in claims 1 and 4. Preferred embodiments are characterized in claims 2, 3, and 5 to 7. In particular,

the absorbent product comprises: a sheet-like main body part where an absorbent core is positioned between a back sheet and a top sheet; and a pair of side wall parts which are provided over almost entire length in a longitudinal direction of the main body part on both side portions of the main body, and in the absorbent product, each of the pair of side wall parts comprises: two fixed parts which are fixed on the main body part in end portions in the longitudinal direction; a standing part between the two fixed parts, being continuous from the two fixed parts and standing from the main body part at a position apart from a side edge of the main body part; a plane part between the two fixed parts, being continuous from the two fixed parts and spreading outward in a width direction of the main body part, the width direction being perpendicular to the longitudinal direction, from an upper end of the standing part, the plane part being thicker than the standing part; and an elastic member for contracting the plane part to form standing gathers, being extended and bonded to the plane part at a position between a center and an outer edge in the width direction, over almost entire length in the longitudinal direction of the plane part. With this structure, it is possible to improve stability of the shape of the plane part in the pair of side wall parts in the absorbent product and to enhance fitting to the wearer.

According to a preferred embodiment of the present invention, each of the pair of side wall parts further comprises another elastic member which is extended and bonded to the plane part at a position between an inner edge and the elastic member, over almost entire length in the longitudinal direction of the plane part.

According to the present invention, the standing part and the plane part are formed of nonwoven fabric, a nonwoven fabric forming the standing part is continuous with a first nonwoven fabric layer of the plane part, and the plane part is a nonwoven fabric-laminated body where a second nonwoven fabric layer is laminated on the first nonwoven fabric layer. It is thereby possible to easily form the plane part.

Preferably, the elastic member is bonded to the outer edge of the plane part, each of the pair of side wall parts further comprises another elastic member which is extended and bonded in the plane part at a position between an inner edge and the outer edge, over almost entire length in the longitudinal direction of the plane part, and a layer over another elastic member of the plane part is thicker than a layer under another elastic member. It is thereby possible to provide the wearer with comfortable feeling in wearing the absorbent product.

More preferably, the second nonwoven fabric layer of the plane part is located under the first nonwoven fabric layer, the second nonwoven fabric layer and the first nonwoven fabric layer are continuous at the outer edge and are not bonded, and another elastic member is bonded only to the second nonwoven fabric layer at a position apart from the outer edge of the plane part.

According to still another preferred embodiment of the present invention, the second nonwoven fabric layer of the plane part is located under the first nonwoven fabric layer, and the second nonwoven fabric layer and the first nonwoven fabric layer are continuous at the outer edge.

According to still another preferred embodiment of the present invention, the elastic member is bonded at a position apart from the outer edge of the plane part, and a layer over the elastic member of the plane part is thicker than a layer under the elastic member.

These and other objects, features, aspects and advantages of the present invention will become more apparent from the following detailed description of the present invention when taken in conjunction with the accompanying drawings.
Fig. 1 is a plan view of an absorbent product in accordance with the first preferred embodiment;
Figs. 2 and 3 are cross-sectional views of the absorbent product;
Fig. 4 is a cross-sectional view of an absorbent product in accordance with the second preferred embodiment; and
Figs. 5, 6 and 7A to 7C are cross-sectional views each showing another example of an absorbent product.

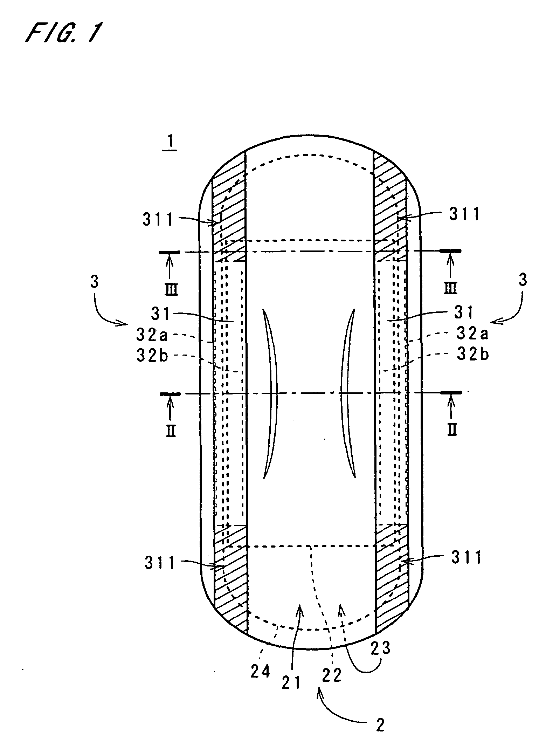

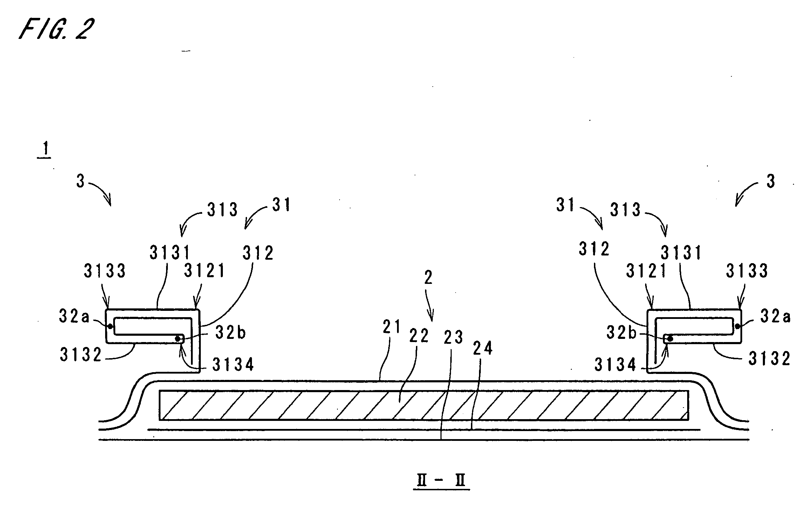

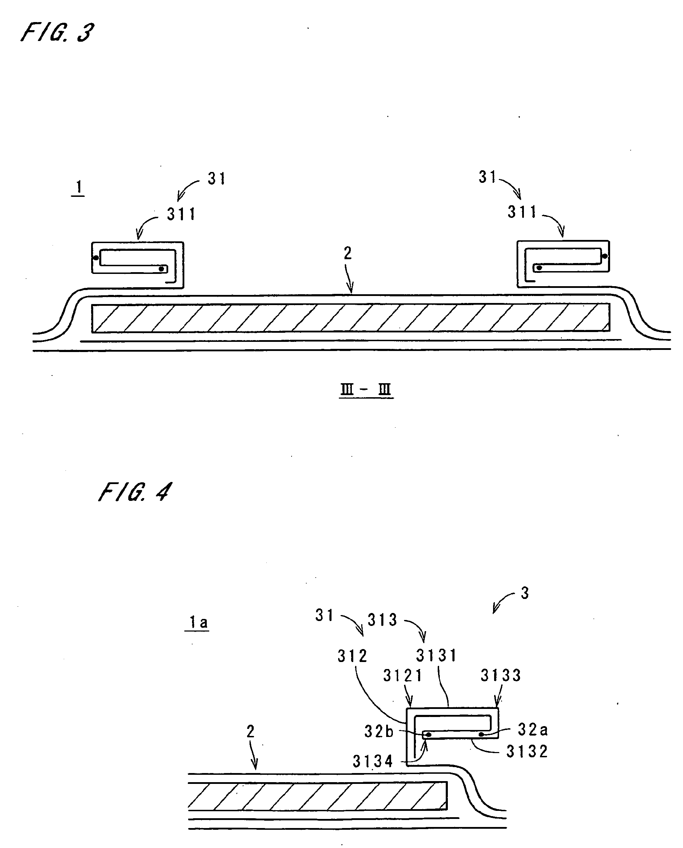

Fig. 1 is a plan view showing an absorbent product 1 in accordance with the first preferred embodiment of the present invention, and Figs. 2 and 3 are cross-sectional views of the absorbent product 1 taken along planes perpendicular to a longitudinal direction (i.e., vertical direction in Fig. 1) of the absorbent product 1 at the positions indicated by the arrows II-II and III-III shown in Fig. 1. The absorbent product 1 is a light incontinence pad for receiving excrement such as urine from a wearer on an inner side of underpants which is an exterior product of the wearer. Fig. 1 shows a wearer's side (i.e., a top side) of the absorbent product 1, which is a side contacting a wearer.

As shown in Figs. 1 and 2, the absorbent product 1 has a sheet-like main body part 2 and a pair of side wall parts 3 which are provided over almost entire length in the longitudinal direction of the main body part 2 on both sides in a width direction perpendicular to the longitudinal direction (i.e., on both side portions of the main body part 2). In the absorbent product 1, the side wall parts 3 stand up toward the wearer on the both sides of the main body part 2 by contracting elastic members provided in the side wall parts 3, to form standing gathers which come into contact with the vicinity of wearer's crotch in wearing.

The main body part 2 has a back sheet 23, a deodorant sheet 24, an absorbent core 22, and a top sheet 21 which are laminated in this order from a side opposed to the wearer's side (i.e., from a lower side). The top sheet 21 covers an upper surface of the absorbent core 22 which is an absorbent, and the back sheet 23 covers a lower surface of the absorbent core 22. In other words, the absorbent core 22 is positioned between the back sheet 23 and the top sheet 21. The top sheet 21 and the back sheet 23 are bonded around the absorbent core 22 and the deodorant sheet 24 by hot melt adhesive or the like, and the side wall parts 3 are bonded on both sides of the absorbent core 22. In Fig. 2, each constituent element of the main body part 2 and the side wall parts 3 are shown separately for convenience of illustration (the same is applied in Figs. 3 to 6 and Figs. 7A to 7C).

The top sheet 21 is a nonwoven fabric made of liquid-pervious sheet material, for example, hydrophilic fiber, and the top sheet 21 immediately catches moisture of excrement from the wearer and moves the moisture into the absorbent core 22. Examples of the nonwoven fabric used for the top sheet 21 are a point-bond nonwoven fabric, air-through nonwoven fabric, or spunlace nonwoven fabric, and as hydrophilic fibers for making these nonwoven fabrics, normally, cellulose, rayon, cotton or the like are used. As the top sheet 21, a liquid-pervious nonwoven fabric made of hydrophobic fiber (for example, polypropylene, polyethylene, polyester, polyamide, or nylon) with hydrophilic treatment using a surfactant may be utilized or a microporous plastic film may be used.

The absorbent core 22 is formed by wrapping a mixture of hydrophilic fibers (e.g., crushed pulp fibers or cellulose fibers) and granulated absorbent polymers (e.g., SAP (Super Absorbent Polymer)) in a cover sheet such as a tissue paper or a liquid-pervious nonwoven fabric, and the absorbent core 22 rapidly absorbs and retains moisture which has passed through the top sheet 21. The cover sheet is bonded to the hydrophilic fibers and the absorbent polymers with the hot melt adhesive, to prevent deformation of the hydrophilic fibers and falling of the absorbent polymers (especially, falling after absorption of moisture).

The back sheet 23 is a water-repellent or liquid-impervious plastic film, and the back sheet 23 prevents the excrement which has passed through the top sheet 21 from leaking outside the main body part 2. From the view point of preventing sweatiness inside the absorbent product 1 and providing comfortable feeling to wearer, it is preferable a plastic film with permeability (i.e., breathability) is used as the back sheet 23. As the back sheet 23, a water-repellent or liquid-impervious nonwoven fabric (e.g., a spunbond nonwoven fabric, a meltblown nonwoven fabric, or a SMS nonwoven fabric) or a laminated sheet where a water-repellent or liquid-impervious plastic film is laminated on an inner side of the water-repellent or liquid-impervious nonwoven fabric can be used.

The deodorant sheet 24 is a hydrophilic sheet member including deodorant agent and for example, cellulose or pulp is used as hydrophilic fibers forming the deodorant sheet 24. As the deodorant agent, inorganic porous crystal such as zeolite which preferably supports metal (e.g., silver, copper, zinc, iron, nickel-cobalt alloy, palladium, platinum) is used.

Each of the pair of side wall parts 3 has a side wall main body 31 formed of a water-repellent or liquid-impervious nonwoven fabric and two elastic members 32a, 32b which are extended in the longitudinal direction and bonded to a later-discussed plane part 313 of the side wall main body 31. In the present preferred embodiment, an elastic member other than the two elastic members 32a, 32b is not provided in the side wall part 3. As a nonwoven fabric forming the side wall main body 31, for example, a spunbond nonwoven fabric, a meltblown nonwoven fabric, or a SMS nonwoven fabric are used. A polyurethane yarn, a strip-like polyurethane film, a yarn-like or strip-like natural rubber, or the like are used as the elastic members 32a, 32b, for example. In the present preferred embodiment, a polyurethane yarn is bonded to the side wall main body 31 by the hot melt adhesive.

The side wall main body 31 has two fixed parts 311 which are bonded and fixed by heat sealing on the main body part 2 in end portions in the longitudinal direction, as shown in Fig. 3. In Fig. 1, each fixed part 311 of the side wall main body 31 is hatched for ease of understanding. The fixed part 311 may be fixed on the main body part 2 with use of ultrasonic bonding or the hot melt adhesive.

As shown in Fig. 2, the side wall main body 31 has a standing part 312 which stands from the main body part 2 at a position apart from a side edge of the main body part 2 toward the wearer (i.e., the position is an inner position than the side edge of the main body part 2) and the plane part 313 which spreads outward in the width direction of the main body part 2 from an upper end 3121 in Fig. 2 of the standing part 312. The standing part 312 and the plane part 313 are located along the longitudinal direction between the two fixed parts 311 (see Fig. 1) (i.e., located in the vicinity of the central portion of the side wall main body 31 in the longitudinal direction), and are continuous to the two fixed parts 311. In the present preferred embodiment, the standing part 312 stands from the main body part 2 at a position apart from a side edge of the absorbent core 22 in the main body part 2 toward the wearer.

The standing part 312 and the plane part 313 are formed of a continuous nonwoven fabric folded into two, and the standing part 312 is formed of two layers of nonwoven fabrics. The plane part 313 has a first nonwoven fabric layer 3131 having two layers of nonwoven fabrics which extend outward in the width direction at the upper end portion 3121 of the standing part 312 and a second nonwoven fabric layer 3132 having two layers of nonwoven fabrics which are formed by folding the first nonwoven fabric layer 3131 which is continuous from the standing part 312, at an outer edge in the width direction of the first nonwoven fabric layer 3131 (i.e., at an outer edge 3133 of the plane part 313), on a lower side and an inner side in the width direction. In other words, the plane part 313 is a nonwoven fabric-laminated body having four layers of nonwoven fabrics where the second nonwoven fabric layer 3132 is laminated on the first nonwoven fabric layer 3131, and the second nonwoven fabric layer 3132 located under the first nonwoven fabric layer 3131 and the first nonwoven fabric layer 3131 are continuous at the outer edge 3133. In the side wall main body 31 of the side wall part 3, since the plane part 313 is formed of four layers of nonwoven fabrics and the standing part 312 is formed of two layers of nonwoven fabrics as described above, the plane part 313 is thicker than the standing part 312. In the side wall main body 31, there may be a case where an edge of the two-folded nonwoven fabric forming the standing part 312 and the plane part 313 is located close to the end portion 3121 of the standing part 312 and the standing part 312 is thereby formed of one layer of nonwoven fabric.

In the plane part 313, the second nonwoven fabric layer 3132 overlaps with almost whole of the first nonwoven fabric layer 3131 in the width direction, and an inner edge 3134 which is an inner end portion in the width direction (i.e., an end portion on the side of the standing part 312) of the second nonwoven fabric layer 3132 is located close to the end portion 3121 of the standing part 312. In the present preferred embodiment, the first nonwoven fabric layer 3131 and the second nonwoven fabric layer 3132 are not bonded, and a small space is provided between the first nonwoven fabric layer 3131 and the second nonwoven fabric layer 3132.

As shown in Figs. 1 and 2, the elastic member 32a is bonded to the outer edge 3133 of the plane part 313, between the two layers of nonwoven fabrics over almost entire length in the longitudinal direction of the plane part 313 (i.e., the outer edge 3133 is a border portion between the first nonwoven fabric layer 3131 and the second nonwoven fabric layer 3132 and is also an outer edge of the first nonwoven fabric layer 3131 and the second nonwoven fabric layer 3132). The elastic member 32b is bonded in the vicinity of the inner edge 3134 of the second nonwoven fabric layer 3132 of the plane part 313, between the two layers of nonwoven fabrics forming the second nonwoven fabric layer 3132 over almost entire length in the longitudinal direction of the plane part 313. In other words, the elastic member 32b is bonded only to the second nonwoven fabric layer 3132 at a position which is between the outer edge 3133 and the inner edge 3134 of the plane part 313 (i.e., a position apart from the outer edge 3133) and is an inner position than the elastic member 32a (i.e., a position between the inner edge 3134 and the elastic member 32a).

In the plane part 313, since the elastic member 32b is sandwiched by the upper three layers of nonwoven fabrics (i.e., the two layers of nonwoven fabrics included in the first nonwoven fabric layer 3131 and one nonwoven fabric of the second nonwoven fabric layer 3132, one nonwoven fabric being close to the first nonwoven fabric layer 3131) and the lower one layer of nonwoven fabric (i.e., the other nonwoven fabric of the second nonwoven fabric layer 3132, the other nonwoven fabric being located under the above one nonwoven fabric), the upper three layers over the elastic member 32b are thicker than the lower one layer under the elastic member 32b.

When the absorbent product 1 shown in Fig. 1 is worn by the wearer, the elastic members 32a, 32b which are extended and bonded to the plane part 313 in each side wall part 3 are contracted, the plane part 313 is thereby contracted between the two fixed parts 311 to form standing gathers, and one surface of the standing gathers (i.e., the first nonwoven fabric layer 3131 in the plane part 313 shown in Fig. 2) comes to be opposed to the vicinity of the wearer's crotch. Then, the plane part 313 deforms along the shape of a portion close to the crotch and the first nonwoven fabric layer 3131 contacts the vicinity of the crotch.

As discussed above, since each of the pair of side wall parts 3 contacts the wearer in the wide plane part 313 which is provided in the end portion 3121 of the standing part 312, even if excrement such as urine reaches the end portion 3121 of the standing part 312, it is possible to prevent movement of the excrement to the outside of the plane part 313 and leakage of the excrement from the absorbent product 1. In each of the side wall parts 3 in the absorbent product 1, the plane part 313 of the side wall main body 31 is thicker than of the standing part 312, to thereby improve stability of the shape of the plane part 313 and enhance fitting of the plane part 313 to the wearer. As a result, it is possible to prevent leakage of excrement from the side wall parts 3 (so-called side leakage) more reliably.

In the side wall part 3, the second nonwoven fabric layer 3132 is laminated on the first nonwoven fabric layer 3131 formed by the nonwoven fabric which is continuous with the standing part 312, to thereby easily form the plane part 313. Since the second nonwoven fabric layer 3132 is formed by folding the nonwoven fabric which is continuous with the first nonwoven fabric layer 3131, on the lower side and the inner side in the width direction, it is possible to form the plane part 313 more easily.

In the side wall part 3, since the elastic member 32b is bonded to a portion between the outer edge 3133 and the inner edge 3134 of the plane part 313 (i.e., between the elastic member 32a and the inner edge 3134), it is possible to improve stability of the shape of the plane part 313 and to further enhance fitting of the plane part 313 to the wearer, as compared with the case where only the elastic member 32a is bonded to the plane part 313.

In the plane part 313, the upper three layers over the elastic member 32b are thicker than the lower one layer under the elastic member 32b and therefore, it is possible to prevent the wearer from being strongly compressed by the elastic member 32b and to provide the wearer with comfortable feeling in wearing the absorbent product 1. At a position apart from the outer edge 3133 of the plane part 313, since no elastic member is bonded to the first nonwoven fabric layer 3131 and the elastic member 32b is bonded only to the second nonwoven fabric layer 3132, flexibility of the first nonwoven fabric layer 3131 is improved and compression to the wearer by the elastic member 32b is decreased to further provide the wearer with comfortable feeling in wearing the absorbent product 1. Furthermore, since the second nonwoven fabric layer 3132 where the elastic member 32b is bonded is not bonded with the first nonwoven fabric layer 3131 to come into contact with the wearer, it is possible to further improve flexibility of the first nonwoven fabric layer 3131 and more decrease compression by the elastic member 32b, to thereby further provide the wearer with comfortable feeling in wearing the absorbent product 1.

In the plane part 313, the elastic member 32b is bonded to the second nonwoven fabric layer 3132 and therefore, it is possible to prevent the second nonwoven fabric layer 3132 which is not bonded with the first nonwoven fabric layer 3131, from hanging out from the first nonwoven fabric layer 3131 and being apart from the first nonwoven fabric layer 3131 more than necessary. As a result, it is possible to further improve stability of the shape of the plane part 313 and to further enhance fitting of the plane part 313 to the wearer. Since the second nonwoven fabric layer 3132 overlaps with almost whole of the first nonwoven fabric layer 3131 in the width direction and the elastic member 32b is located in the inner edge 3134 which is an end portion on the side of the standing part 312 of the second nonwoven fabric layer 3132, almost whole of the plane part 313 is thicker than the standing part 312 and both edge portions in the width direction of the plane part 313 are supported by the elastic members 32a, 32b. As a result, it is possible to further improve stability of the shape of the plane part 313 and to further enhance fitting of the plane part 313 to the wearer.

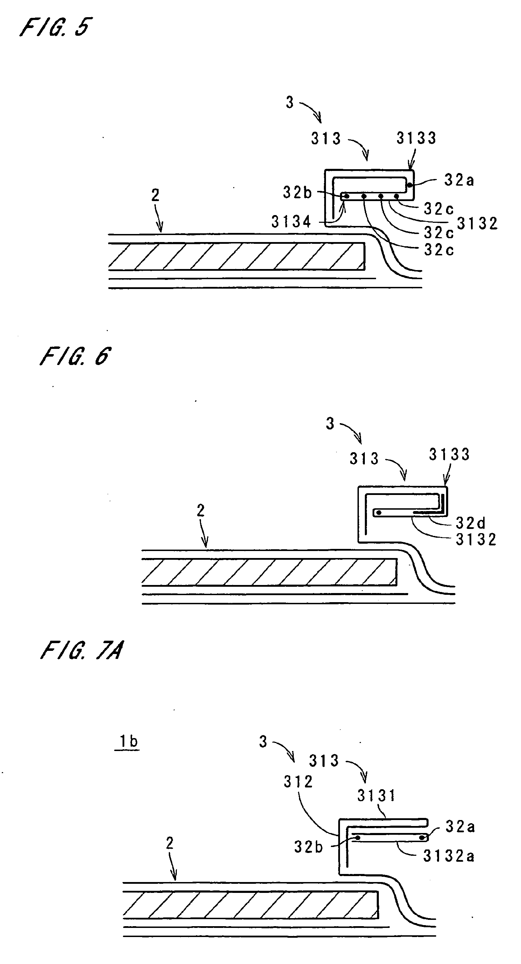

Next discussion will be made on an absorbent product in accordance with the second preferred embodiment of the present invention. Fig. 4 is a cross-sectional view showing a part of an absorbent product 1a according to the second preferred embodiment. Fig. 4 shows a right part of a cross section of the absorbent product 1a at positions corresponding to the positions indicated by the arrows II-II in Fig. 1, and a left part (not shown) of the cross section of the absorbent product 1a also has the same structure as the right part (the same is applied in Figs. 5 to 7C). As shown in Fig. 4, in the absorbent product 1a, a position where an elastic member 32a is bonded in the plane part 313 of the side wall part 3 is different from that in the absorbent product 1 shown in Fig. 2. The other constituent elements are the same as those in the first preferred embodiment and represented by the same reference signs.

As shown in Fig. 4, in each of the side wall parts 3 in the absorbent product 1a, the elastic member 32a is bonded at a position which is between the center (i.e., the center line between the inner edge 3134 and the outer edge 3133 of the plane part 313) and the outer edge 3133 in the width direction of the plane part 313. The elastic member 32a is bonded between the two layers of nonwoven fabrics forming the second nonwoven fabric layer 3132 over almost entire length in the longitudinal direction of the plane part 313. Similarly to the first preferred embodiment, the elastic member 32b is bonded in the vicinity of the inner edge 3134 of the second nonwoven fabric layer 3132 of the plane part 313, between the two layers of nonwoven fabrics forming the second nonwoven fabric layer 3132 over almost entire length in the longitudinal direction of the plane part 313. That is, in the plane part 313, the two elastic members 32a, 32b are bonded only to the second nonwoven fabric layer 3132 at a position apart from the outer edge 3133, and no elastic member is bonded to the first nonwoven fabric layer 3131 which is not bonded with the second nonwoven fabric layer 3132.

In the plane part 313, similarly to the elastic member 32b in the absorbent product 1 according to the first preferred embodiment, since the elastic members 32a, 32b are sandwiched by the upper three layers of nonwoven fabrics and the lower one layer of nonwoven fabric, the upper three layers over the elastic member 32a are thicker than the lower one layer under the elastic member 32a and the upper three layers over the elastic member 32b are thicker than the lower one layer under the elastic member 32b.

In the absorbent product 1a according to the second preferred embodiment, similarly to the first preferred embodiment, since each of the pair of side wall parts 3 contacts the wearer in the wide plane part 313 which is provided in the end portion 3121 of the standing part 312, it is possible to prevent leakage of excrement from the side wall parts 3 (so-called side leakage). In each of the side wall parts 3, the plane part 313 of the side wall main body 31 is thicker than the standing part 312, to thereby improve stability of the shape of the plane part 313 and enhance fitting of the plane part 313 to the wearer. As a result, it is possible to prevent leakage of excrement from the side wall parts 3 more reliably.

In the side wall part 3, the second nonwoven fabric layer 3132 is laminated on the first nonwoven fabric layer 3131 formed by the nonwoven fabric which is continuous with the standing part 312, to thereby easily form the plane part 313. Since the second nonwoven fabric layer 3132 is formed by folding the nonwoven fabric which is continuous with the first nonwoven fabric layer 3131, at the outer edge 3133 of the plane part 313, on the lower side and the inner side in the width direction, it is possible to form the plane part 313 more easily.

In the plane part 313, the upper three layers over the elastic members 32a, 32b are thicker than the lower one layer under the elastic members 32a, 32b and therefore, it is possible to prevent the wearer from being strongly compressed by the elastic members 32a, 32b and to provide the wearer with comfortable feeling in wearing the absorbent product 1a. At a position apart from the outer edge 3133 of the plane part 313, since no elastic member is bonded to the first nonwoven fabric layer 3131 and the elastic members 32a, 32b are bonded only to the second nonwoven fabric layer 3132, flexibility of the first nonwoven fabric layer 3131 is improved and compression by the elastic members 32a, 32b is decreased to further provide the wearer with comfortable feeling in wearing the absorbent product 1a. Furthermore, since the second nonwoven fabric layer 3132 where the elastic members 32a, 32b are bonded is not bonded with the first nonwoven fabric layer 3131 to come into contact with the wearer, it is possible to further improve flexibility of the first nonwoven fabric layer 3131 and more decrease compression by the elastic members 32a, 32b, to thereby further provide the wearer with comfortable feeling in wearing the absorbent product 1a.

Though the preferred embodiments of the present invention have been discussed above, the present invention is not limited to the above-discussed preferred embodiments, but allows various variations.

In the side wall part 3 in the absorbent products according to the above preferred embodiments, the width of the second nonwoven fabric layer 3132 may be much smaller than that of the first nonwoven fabric layer 3131. For example, in a case where the width of the second nonwoven fabric layer 3132 is about half that of the first nonwoven fabric layer 3131 and the second nonwoven fabric layer 3132 is not apart from the first nonwoven fabric layer 3131 more than necessary, the elastic member 32a is provided between the center and the outer edge 3133 of the plane part 313 in the width direction and the elastic member 32b may be omitted. As described, even in the case that only one elastic member 32a is provided in the plane part 313, it is possible to improve stability of the shape of the plane part 313 by making the plane part 313 thicker than the standing part 312.

As shown in Fig. 5, two or more elastic members extending in the longitudinal direction (in Fig. 5, one elastic member 32b provided in the inner edge 3134 of the second nonwoven fabric layer 3132 and three elastic members 32c arranged between the elastic members 32a and 32b) may be provided in the plane part 313, other than the elastic member 32a provided in the outer edge 3133. As shown in Fig. 6, a strip-like elastic member 32d which spreads from the outer edge 3133 in the plane part 313 to between the two layers of nonwoven fabrics of the second nonwoven fabric layer 3132, may be provided in the plane part 313.

In the side wall part 3, the first nonwoven fabric layer 3131 and the second nonwoven fabric layer 3132 in the plane part 313 are not necessarily formed by a continuous nonwoven fabric, but for example, like in an absorbent product 1b shown in Fig. 7A, the plane part 313 may be formed by bonding a second nonwoven fabric layer 3132a below the first nonwoven fabric layer 3131 which is continuous with the standing part 312, the second nonwoven fabric layer 3132a being formed by two layers of nonwoven fabrics (i.e., a nonwoven fabric folded into two) and being independently of the first nonwoven fabric layer 3131.

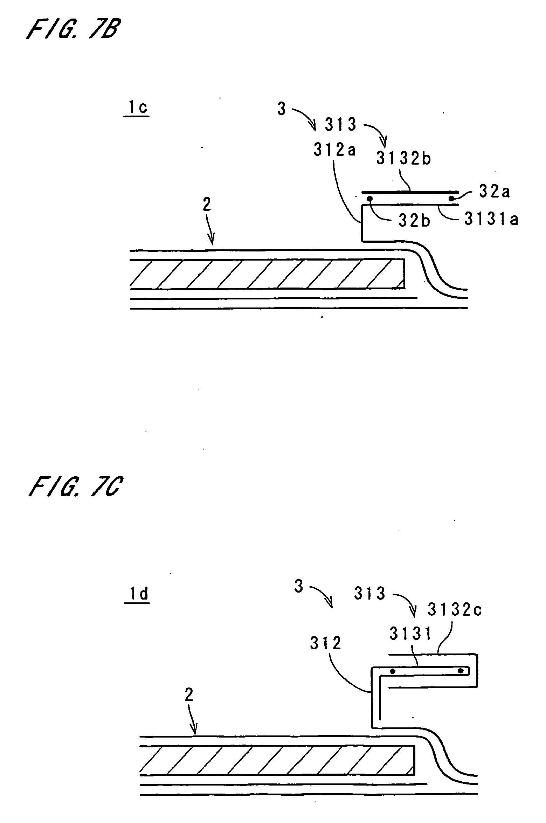

Like in an absorbent product 1c shown in Fig. 7B, there may be a case where a standing part 312a and a first nonwoven fabric layer 3131 a continuous with the standing part 312a are formed by one layer of nonwoven fabric, and a second nonwoven fabric layer 3132b formed by one layer of nonwoven fabric which is thicker than the first nonwoven fabric layer 3131 a is bonded above the first nonwoven fabric layer 3131a with interposing the elastic members 32a, 32b, to thereby form the plane part 313.

In both cases of the absorbent product 1b and the absorbent product 1c shown in Figs. 7A and 7B, since the plane part 313 is thicker than the standing part in each side wall part 3, it is possible to improve stability of the shape of the plane part 313 and enhance fitting of the plane part 313 to the wearer and as a result, leakage of excrement from the side wall part 3 is prevented more reliably. Since the layer(s) over the elastic members 32a, 32b is thicker than the layer(s) under the elastic members 32a, 32b, it is possible to prevent the wearer from being strongly compressed by the elastic members 32a, 32b and to provide the wearer with comfortable feeling in wearing the absorbent products 1b, 1c. In the absorbent product 1b shown in Fig. 7A, the second nonwoven fabric layer 3132a and the elastic members 32a, 32b may be bonded above the first nonwoven fabric layer 3131 and also in this case, stability of the shape of the plane part 313 can be improved.

Like in an absorbent product 1d shown in Fig. 7C, the plane part 313 may be formed by bonding a second nonwoven fabric layer 3132c with the first nonwoven fabric layer 3131, the second nonwoven fabric layer 3132c being one nonwoven fabric which is folded into two so as to cover above and below the first nonwoven fabric layer 3131. Also in this case, since the plane part 313 is thicker than the standing part 312 in each side wall part 3, it is possible to improve stability of the shape of the plane part 313 and enhance fitting of the plane part 313 to the wearer.

The side wall main body 31 is not necessarily formed by a nonwoven fabric but may be formed by, e.g., a water-repellent or liquid-impervious plastic film or a laminated sheet of a water-repellent or liquid-impervious nonwoven fabric and a plastic film. By using a plastic film with permeability (i.e., breathability) for the side wall main body 31, it is possible to prevent sweatiness inside the absorbent product and to provide comfortable feeling to wearer.

The structure of the absorbent products according to the above preferred embodiments may be applied to various absorbent products such as a disposal diaper, an auxiliary absorbent pad which is attached on an inner side of a disposal diaper, a sanitary napkin, and a panty liner, as well as a light incontinence pad.

While the invention has been shown and described in detail, the foregoing description is in all aspects illustrative and not restrictive. It is therefore understood that numerous modifications and variations can be devised without departing from the scope of the invention.

## Claims

1. An absorbent product (1, 1 a to 1 d) for receiving excrement from a wearer, comprising:
a sheet-like main body part (2) where an absorbent core (22) is positioned between a back sheet (23) and a top sheet (21); and
a pair of side wall parts (3) which are provided over almost entire length in a longitudinal direction of said main body part (2) on both side portions of said main body (2), wherein
each of said pair of side wall parts (3) comprises:
two fixed parts (311) which are fixed on said main body part (2) in end portions in said longitudinal direction;
a standing part (312, 312a) between said two fixed parts (311), being continuous from said two fixed parts (311) and standing from said main body part (2) at a position apart from a side edge of said main body part (2);
a plane part (313) between said two fixed parts (311), being continuous from said two fixed parts (311) and spreading outward in a width direction of said main body part (2), said width direction being perpendicular to said longitudinal direction, from an upper end (3121) of said standing part (312, 312a); and
an elastic member (32a, 32d) for contracting said plane part (313) to form standing gathers, being extended and bonded to an outer edge (3133) of said plane part (313) in said width direction, over almost entire length in said longitudinal direction of said plane part (313), and **characterized in that** said plane part (313) is thicker than said standing part (312, 312a), and wherein
said standing part (312, 312a) and said plane part (313) are formed of nonwoven fabric,
a nonwoven fabric forming said standing part (312, 312a) is continuous with a first nonwoven fabric layer (3131, 3131 a) of said plane part (313), and
said plane part (313) is a nonwoven fabric-laminated body where a second nonwoven fabric layer (3132, 3132a to 3132c) is laminated on said first nonwoven fabric layer (3131, 3131 a), and wherein
said elastic member (32a, 32d) is bonded to said outer edge (3133) of said plane part (313),
each of said pair of side wall parts (3) further comprises another elastic member (32b, 32c) which is extended and bonded in said plane part (313) at a position between an inner edge (3134) and said outer edge (3133), over almost entire length in said longitudinal direction of said plane part (313), and
a layer over said another elastic member (32c) of said plane part (313) is thicker than a layer under said another elastic member (32c).

2. The absorbent product (1, 1a to 1d) according to claim 1, wherein said second nonwoven fabric layer (3132) of said plane part (313) is located under said first nonwoven fabric layer (3131, 3131a), and
said second nonwoven fabric layer (3132) and said first nonwoven fabric layer (3131, 3131a) are continuous at said outer edge (3133).

3. The absorbent product (1, 1a to 1d) according to claim 2, wherein
said second nonwoven fabric layer (3132) and said first nonwoven fabric layer (3131, 3131a) are not bonded, and
said another elastic member (32b, 32c) is bonded only to said second nonwoven fabric layer (3132) at a position apart from said outer edge (3133) of said plane part (313).

4. An absorbent product (1, 1a to 1d) for receiving excrement from a wearer, comprising:
a sheet-like main body part (2) where an absorbent core (22) is positioned between a back sheet (23) and a top sheet (21); and
a pair of side wall parts (3) which are provided over almost entire length in a longitudinal direction of said main body part (2) on both side portions of said main body (2), wherein
each of said pair of side wall parts (3) comprises:
two fixed parts (311) which are fixed on said main body part (2) in end portions in said longitudinal direction;
a standing part (312, 312a) between said two fixed parts (311), being continuous from said two fixed parts (311) and standing from said main body part (2) at a position apart from a side edge of said main body part (2);
a plane part (313) between said two fixed parts (311), being continuous from said two fixed parts (311) and spreading outward in a width direction of said main body part (2), said width direction being perpendicular to said longitudinal direction, from an upper end (3121) of said standing part (312, 312a); and
an elastic member (32a, 32d) for contracting said plane part (313) to form standing gathers, being extended and bonded to said plane part (313) at a position between a center and an outer edge (3133) in said width direction, over almost entire length in said longitudinal direction of said plane part (313), and **characterized in that** said plane part (313) is thicker than said standing part (312, 312a), and wherein
said standing part (312, 312a) and said plane part (313) are formed of nonwoven fabric,
a nonwoven fabric forming said standing part (312, 312a) is continuous with a first nonwoven fabric layer (3131, 3131a) of said plane part (313), and
said plane part (313) is a nonwoven fabric-laminated body where a second nonwoven fabric layer (3132, 3132a to 3132c) is laminated on said first nonwoven fabric layer (3131, 3131 a), and wherein
said elastic member (32a, 32d) is bonded at a position apart from said outer edge (3133) of said plane part (313), and
a layer over said elastic member (32a, 32d) of said plane part (313) is thicker than a layer under said elastic member (32a, 32d).

5. The absorbent product (1, 1 a to 1 d) according to claim 4, wherein
said second nonwoven fabric layer (3132) of said plane part (313) is located under said first nonwoven fabric layer (3131, 3131 a), and
said second nonwoven fabric layer (3132) and said first nonwoven fabric layer (3131, 3131 a) are continuous at said outer edge (3133).

6. The absorbent product (1, 1 a to 1 d) according to claim 5, wherein
said first nonwoven fabric layer (3131, 3131 a) and said second nonwoven fabric layer (3132) are not bonded, and
said elastic member (32a, 32d) is bonded only to said second nonwoven fabric layer (3132) at a position apart from said outer edge (3133) of said plane part (313).

7. The absorbent product (1, 1a to 1 d) according to claims 3 or 6, wherein said second nonwoven fabric layer (3132, 3132a to 3132c) overlaps with almost whole of said first nonwoven fabric layer (3131, 3131 a) in said width direction, and
an elastic member (32b) is bonded to an inner edge (3134) of said second nonwoven fabric layer (3132, 3132a to 3132c).

## Patentansprüche

1. Absorbensprodukt (1, 1a bis 1d) zum Aufnehmen von Exkrementen von einem Träger, umfassend:
einen lagenartigen Hauptkörperteil (2), bei dem ein Absorbenskern (22) zwischen einer Rückseitenlage (23) und einer Oberseitenlage (21) angeordnet ist, und
ein Paar von Seitenwandteilen (3), die über nahezu der gesamten Länge in einer Längsrichtung des Hauptkörperteils (2) auf beiden Seitenabschnitten des Hauptkörpers (2) bereitgestellt sind, wobei
jedes des Paars von Seitenwandteilen (3) umfasst:
zwei fixierte Teile (311), die auf dem Hauptkörperteil (2) in Endabschnitten in der Längsrichtung fixiert sind,
einen aufrecht stehenden Teil (312, 312a) zwischen den zwei fixierten Teilen (311), der sich kontinuierlich von den zwei fixierten Teilen (311) erstreckt und von dem Hauptkörperteil (2) an einer Position entfernt von einer Seitenkante des Hauptkörperteils (2) absteht,
einen ebenen Teil (313) zwischen den zwei fixierten Teilen (311), der sich kontinuierlich von den zwei fixierten Teilen (311) erstreckt und sich in einer Breitenrichtung des Hauptkörperteils (2), wobei die Breitenrichtung senkrecht zu der Längsrichtung ist, von einem oberen Ende (3121) des aufrecht stehenden Teils (312, 312a) auswärts erstreckt, und
ein elastisches Element (32a, 32d) zum Zusammenziehen des ebenen Teils (313) zur Bildung von aufrecht stehenden Raffungen, das sich über nahezu der gesamten Länge in der Längsrichtung des ebenen Teils (313) zu einer Außenkante (3133) des ebenen Teils (313) in der Breitenrichtung erstreckt und daran gebunden ist, und **dadurch gekennzeichnet, dass** der ebene Teil (313) dicker ist als der aufrecht stehende Teil (312, 312a) und wobei
der aufrecht stehende Teil (312, 312a) und der ebene Teil (313) aus einem Vlies ausgebildet sind,
wobei ein Vlies, das den aufrecht stehenden Teil (312, 312a) bildet, sich kontinuierlich mit einer ersten Vliesschicht (3131, 3131 a) des ebenen Teils (313) erstreckt und
der ebene Teil (313) ein Vlies-laminierter Körper ist, wobei eine zweite Vliesschicht (3132, 3132a bis 3132c) auf die erste Vliesschicht (3131, 3131a) laminiert ist, und wobei
das elastische Element (32a, 32d) an die Außenkante (3133) des ebenen Teils (313) gebunden ist,
wobei jedes des Paars von Seitenwandteilen (3) ferner ein weiteres elastisches Element (32b, 32c) umfasst, das sich in dem ebenen Teil (313) an einer Position zwischen einer Innenkante (3134) und der Außenkante (3133) über nahezu die gesamte Länge in der Längsrichtung des ebenen Teils (313) erstreckt und daran gebunden ist, und
wobei eine Schicht über dem weiteren elastischen Element (32c) des ebenen Teils (313) dicker ist als eine Schicht unter dem weiteren elastischen Element (32c).

2. Absorbensprodukt (1, 1a bis 1 d) nach Anspruch 1, wobei die zweite Vliesschicht (3132) des ebenen Teils (313) unter der ersten Vliesschicht (3131, 3131 a) angeordnet ist, und
die zweite Vliesschicht (3132) und die erste Vliesschicht (3131, 3131a) an der Au-βenkante (3133) kontinuierlich sind.

3. Absorbensprodukt (1, 1 a bis 1 d) nach Anspruch 2, wobei die zweite Vliesschicht (3132) und die erste Vliesschicht (3131, 3131a) nicht gebunden sind, und
das weitere elastische Element (32b, 32c) an einer Position entfernt von der Außenkante (3133) des ebenen Teils (313) nur an die zweite Vliesschicht (3132) gebunden ist.

4. Absorbensprodukt (1, 1a bis 1 d) zum Aufnehmen von Exkrementen von einem Träger, umfassend:
einen lagenartigen Hauptkörperteil (2), bei dem ein Absorbenskern (22) zwischen einer Rückseitenlage (23) und einer Oberseitenlage (21) angeordnet ist, und
ein Paar von Seitenwandteilen (3), die über nahezu der gesamten Länge in einer Längsrichtung des Hauptkörperteils (2) auf beiden Seitenabschnitten des Hauptkörpers (2) bereitgestellt sind, wobei
jedes des Paars von Seitenwandteilen (3) umfasst:
zwei fixierte Teile (311), die auf dem Hauptkörperteil (2) in Endabschnitten in der Längsrichtung fixiert sind,
einen aufrecht stehenden Teil (312, 312a) zwischen den zwei fixierten Teilen (311), der sich kontinuierlich von den zwei fixierten Teilen (311) erstreckt und von dem Hauptkörperteil (2) an einer Position entfernt von einer Seitenkante des Hauptkörperteils (2) absteht,
einen ebenen Teil (313) zwischen den zwei fixierten Teilen (311), der sich kontinuierlich von den zwei fixierten Teilen (311) erstreckt und sich in einer Breitenrichtung des Hauptkörperteils (2), wobei die Breitenrichtung senkrecht zu der Längsrichtung ist,
von einem oberen Ende (3121) des aufrecht stehenden Teils (312, 312a) auswärts erstreckt, und
ein elastisches Element (32a, 32d) zum Zusammenziehen des ebenen Teils (313) zur Bildung von aufrecht stehenden Raffungen, das sich an einer Position zwischen einem Zentrum und einer Außenkante (3133) in der Breitenrichtung über nahezu der gesamten Länge in der Längsrichtung des ebenen Teils (313) zu dem ebenen Teil (313) erstreckt und daran gebunden ist, und **dadurch gekennzeichnet, dass** der ebene Teil (313) dicker ist als der aufrecht stehende Teil (312, 312a) und
wobei der aufrecht stehende Teil (312, 312a) und der ebene Teil (313) aus einem Vlies ausgebildet sind,
wobei ein Vlies, das den aufrecht stehenden Teil (312, 312a) bildet, sich kontinuierlich mit einer ersten Vliesschicht (3131, 3131 a) des ebenen Teils (313) erstreckt und
der ebene Teil (313) ein Vlies-laminierter Körper ist, wobei eine zweite Vliesschicht (3132, 3132a bis 3132c) auf die erste Vliesschicht (3131, 3131 a) laminiert ist, und wobei
das elastische Element (32a, 32d) an einer Position entfernt von der Außenkante (3133) des ebenen Teils (313) gebunden ist, und
wobei eine Schicht über dem elastischen Element (32a, 32d) des ebenen Teils (313) dicker ist als eine Schicht unter dem elastischen Element (32a, 32d).

5. Absorbensprodukt (1, 1 a bis 1 d) nach Anspruch 4, wobei die zweite Vliesschicht (3132) des ebenen Teils (313) unter der ersten Vliesschicht (3131, 3131 a) angeordnet ist, und
die zweite Vliesschicht (3132) und die erste Vliesschicht (3131, 3131a) an der Au-βenkante (3133) kontinuierlich sind.

6. Absorbensprodukt (1, 1a bis 1d) nach Anspruch 5, wobei
die erste Vliesschicht (3131, 3131a) und die zweite Vliesschicht (3132) nicht gebunden sind, und
das elastische Element (32a, 32d) an einer Position entfernt von der Außenkante (3133) des ebenen Teils (313) nur an die zweite Vliesschicht (3132) gebunden ist.

7. Absorbensprodukt (1, 1a bis 1d) nach Anspruch 3 oder 6, wobei die zweite Vliesschicht (3132, 3132a bis 3132c) mit nahezu der gesamten ersten Vliesschicht (3131, 3131 a) in der Breitenrichtung überlappt, und
ein elastisches Element (32b) an eine Innenkante (3134) der zweiten Vliesschicht (3132, 3132a bis 3132c) gebunden ist.

## Revendications

1. Un produit absorbant (1, 1a à 1d) destiné à recevoir les excréments d'un porteur, comprenant :
une partie de corps principal ayant l'aspect d'une feuille (2) dans laquelle un coeur absorbant (22) est positionné entre une feuille inférieure (23) et une feuille supérieure (21) ; et
une paire de parties de paroi latérale (3) qui sont présentes sur pratiquement toute la longueur dans le sens longitudinal de ladite partie de corps principal (2) sur les deux portions latérales dudit corps principal (2), dans lequel
chacune de ladite paire de parties de paroi latérale (3) comprend :
deux parties fixes (311) qui sont fixées sur ladite partie de corps principal (2) dans des portions terminales dans ledit sens longitudinal ;
une partie relevée (312, 312a) entre lesdites deux parties fixes (311), qui est continue depuis lesdites deux parties fixes (311) et se relevant depuis ladite partie de corps principal (2) en une position séparée d'un bord latéral de ladite partie de corps principal (2) ;
une partie plane (313) entre lesdites deux parties fixes (311), qui est continue depuis lesdites deux parties fixes (311) et s'étendant vers l'extérieur dans le sens de la largeur de ladite partie de corps principal (2), ledit sens de la largeur étant perpendiculaire audit sens longitudinal, depuis une extrémité supérieure (3121) de ladite partie relevée (312, 312a) ; et
un élément élastique (32a, 32d) destiné à contracter ladite partie plane (313) afin de former des fronces relevées, qui s'étendent et sont fixées sur un bord extérieur (3133) de ladite partie plane (313 dans ledit sens de la largeur, sur la quasi-totalité de la longueur dans ledit sens longitudinal de ladite partie plane (313), et **caractérisé en ce que** ladite partie plane (313) est plus épaisse que ladite partie relevée (312, 312a), et dans lequel ladite partie relevée (312, 312a) et ladite partie plane (313) sont formées d'un tissu non tissé,
un tissu non tissé formant ladite partie relevée (312, 312a) est continu avec une première couche de tissu non tissé (3131, 3131a) de ladite partie plane (313), et ladite partie plane (313) est un corps en tissu non tissé laminé avec un tissu où une seconde couche de tissu non tissé (3132, 3132a à 3132c) est laminée sur ladite première couche de tissu non tissé (3131, 3131a), et dans lequel
ledit élément élastique (32a, 32d) est soudé audit bord extérieur (3133) de ladite partie plane (313),
chacune de ladite paire de parties de paroi latérale (3) comprend en outre un autre élément élastique (32b, 32c) qui s'étend et est fixé sur ladite partie plane (313) en une position entre un bord intérieur (3134) et ledit bord extérieur (3313), sur la quasi-totalité de la longueur dans ledit sens longitudinal de ladite partie plane (313), et
une couche par-dessus ledit autre élément élastique (32c) de ladite partie plane (313) est plus épaisse qu'une couche en-dessous dudit autre élément élastique (32c).

2. Le produit absorbant (1, 1a à 1d) selon la revendication 1, dans lequel ladite deuxième couche de tissu non tissé (3132) de ladite partie plane (313) est située en dessous de ladite première couche de tissu non tissé (3131, 3131a), et
ladite deuxième couche de tissu non tissé (3132) et ladite première couche de tissu non tissé (3131, 3131a) sont continues audit bord extérieur (3133).

3. Le produit absorbant (1, 1a à 1d) selon la revendication 2, dans lequel ladite deuxième couche de tissu non tissé (3132) et ladite première couche de tissu non tissé (3131, 3131a) ne sont pas fixées, et
ledit autre élément élastique (32b, 32c) est fixé uniquement à ladite deuxième couche de tissu non tissé (3132) en une position séparée dudit bord extérieur (3133) de ladite partie plane (313).

4. Un produit absorbant (1, 1a à 1d) destiné à recevoir les excréments d'un porteur, comprenant :
une partie de corps principal ayant l'aspect d'une feuille (2) dans laquelle un coeur absorbant (22) est positionné entre une feuille inférieure (23) et une feuille supérieure (21) ; et
une paire de parties de paroi latérale (3) qui sont présentes sur pratiquement toute la longueur dans le sens longitudinal de ladite partie de corps principal (2) sur les deux portions latérales dudit corps principal (2), dans lequel
chacune de ladite paire de parties de paroi latérale (3) comprend :
deux parties fixes (311) qui sont fixées sur ladite partie de corps principal (2) dans des portions terminales dans ledit sens longitudinal ;
une partie relevée (312, 312a) entre lesdites deux parties fixes (311), qui est continue depuis lesdites deux parties fixes (311) et se relevant depuis ladite partie de corps principal (2) en une position séparée d'un bord latéral de ladite partie de corps principal (2) ;
une partie plane (313) entre lesdites deux parties fixes (311), qui est continue depuis lesdites deux parties fixes (311) et s'étendant vers l'extérieur dans le sens de la largeur de ladite partie de corps principal (2), ledit sens de la largeur étant perpendiculaire audit sens longitudinal, depuis une extrémité supérieure (3121) de ladite partie relevée (312, 312a) ; et
un élément élastique (32a, 32d) destiné à contracter ladite partie plane (313) afin de former des fronces relevées, qui s'étendent et sont fixées sur ladite partie plane (313) en une position entre un centre et un bord extérieur (3133) dans ledit sens de la largeur, sur la quasi-totalité de la longueur dans ledit sens longitudinal de ladite partie plane (313), et **caractérisé en ce que** ladite partie plane (313) est plus épaisse que ladite partie relevée (312, 312a), et dans lequel ladite partie relevée (312, 312a) et ladite partie plane (313) sont formées d'un tissu non tissé,
un tissu non tissé formant ladite partie relevée (312, 312a) est continu avec une première couche de tissu non tissé (3131, 3131a) de ladite partie plane (313), et ladite partie plane (313) est un corps en tissu non tissé laminé avec un tissu où une seconde couche de tissu non tissé (3132, 3132a à 3132c) est laminée sur ladite première couche de tissu non tissé (3131, 3131a), et dans lequel
ledit élément élastique (32a, 32d) est soudé en une position séparée dudit bord extérieur (3133) de ladite partie plane (313), et
une couche par-dessus ledit élément élastique (32a, 32d) de ladite partie plane (313) est plus épaisse qu'une couche en-dessous dudit autre élément élastique (32a, 32d).

5. Le produit absorbant (1, 1a à 1d) selon la revendication 4, dans lequel ladite deuxième couche de tissu non tissé (3132) de ladite partie plane (313) est située en dessous de ladite première couche de tissu non tissé (3131, 3131a), et ladite deuxième couche de tissu non tissé (3132) et ladite première couche de tissu non tissé (3131, 3131a) sont continues audit bord extérieur (3133).

6. Le produit absorbant (1, 1a à 1d) selon la revendication 5, dans lequel ladite première couche de tissu non tissé (3131, 3131a) et ladite deuxième couche de tissu non tissé (3132) ne sont pas fixées, et
ledit élément élastique (32a, 32d) est fixé uniquement à ladite deuxième couche de tissu non tissé (3132) en une position séparée dudit bord extérieur (3133) de ladite partie plane (313)

7. Le produit absorbant (1, 1a à 1d) selon les revendications 3 ou 6, dans lequel ladite deuxième couche de tissu non tissé (3132, 3132a à 3132c) chevauche la quasi-totalité de ladite première couche de tissu non tissé (3131, 3131a) dans ledit sens de la largeur, et
un élément élastique (32b) est fixé à un bord intérieur (3134) de ladite deuxième couche de tissu non tissé (3132, 3132a à 3132c).
